# EUROPEAN PATENT APPLICATION

(11) **EP 4 778 447 A1**
(43) Date of publication of application: **22.07.2026**
(21) Application number: 24865452.7
(22) Date of filing: 10.09.2024
(51) Int. Cl.: A61B 3/113, A61B 3/08, A61B 3/107

(54) **STRABISMUS DETERMINATION DEVICE, STRABISMUS DETERMINATION METHOD, AND PROGRAM**

(30) Priority: 13.09.2023 JP 2023148530
(71) Applicant: Innojin, Inc., Tokyo 113-0033 (JP); Juntendo Educational Foundation, Tokyo 113-8421 (JP)
(72) Inventor: INOMATA, Takenori, Tokyo 113-0033 (JP); OKUMURA, Yuichi, Tokyo 113-0033 (JP)
(74) Representative: Osterhoff, Utz
(86) International application number: PCT/JP2024/032420
(87) International publication number: WO 2025/057952

(57) **Abstract**

[Problem] To enable easy determination of strabismus.

[Solution] A strabismus determination apparatus comprising: an image acquisition unit configured to acquire an image of the eyes of an examinee captured from the front of the examinee; a cornea detection unit configured to analyze the image and detect shapes corresponding to the corneas from the image; and a strabismus determination unit configured to determine a degree of strabismus based on at least one of a width and a height of the corneas.

## Description

### Technical Field

The present invention relates to a strabismus determination apparatus, a strabismus determination method, and a program.

### Background Art

Strabismus is determined by reflecting light from a light source on the eye of an examinee (PTL 1).

### Citation List

### Patent Literature

[PTL1] Japanese Patent Publication No. 2015-525597

### Summary of Invention

### Technical Problem

However, in PTL 1, it is necessary to set a light source and measure reflection of light, which is time-consuming.

The present invention has been made in view of the above circumstances, and an object thereof is to provide a technology capable of easily determining strabismus.

### Solution to Problem

According to one aspect of the present invention, there is provided a strabismus determination apparatus comprising: an image acquisition unit configured to acquire an image captured of the eyes of an examinee from the front of the examinee; a cornea detection unit configured to analyze the image and detect a shape corresponding to the cornea from the image; and a strabismus determination unit configured to determine a degree of strabismus based on at least one of a width and a height of the cornea.

Other objects, features, and advantages of the present application will become apparent from the following description of embodiments and the accompanying drawings.

### Advantageous Effects of Invention

According to the present invention, strabismus can be easily determined.

### Brief Description of Drawings

[Fig. 1] Fig. 1 is a diagram illustrating an image obtained by capturing an eye of an examinee.
[Fig. 2] Fig. 2 is a diagram showing a hardware configuration example of a strabismus determination apparatus 2.
[Fig. 3] Fig. 3 is a diagram showing a software configuration example of the strabismus determination apparatus 2.
[Fig. 4] Fig. 4 is a diagram illustrating a flow of processing by the strabismus determination apparatus 2.
[Fig. 5] Fig. 5 is a diagram illustrating a state of capturing an examinee according to a second embodiment.
[Fig. 6] Fig. 6 is a diagram illustrating a flow of processing by the strabismus determination apparatus 2 according to the second embodiment.

### Description of Embodiments

Hereinafter, a strabismus determination apparatus 2 according to an embodiment of the present invention will be described. The strabismus determination apparatus 2 of the present embodiment is configured to determine a degree of strabismus of an examinee from an image captured of the examinee.

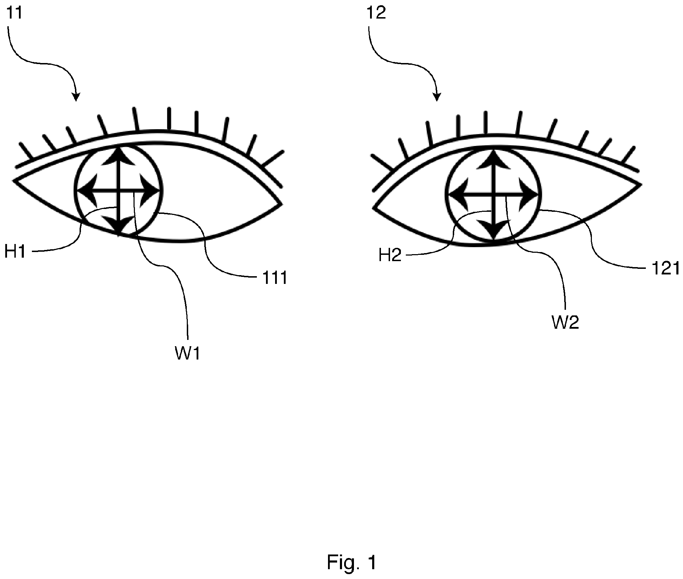

Fig. 1 is a diagram for explaining an image captured of eyes of an examinee. In the present embodiment, it is assumed that the examinee is captured from the front using the strabismus determination apparatus 2. In the example of Fig. 1, a state of so-called strabismus in which a right eye 11 is not looking straight ahead is shown.

A left eye 12 is looking straight ahead, and in this case, a cornea 121 of the left eye 12 has an approximately circular shape. That is, a width W2 and a height H2 of the cornea 121 are approximately the same.

On the other hand, the right eye 11 is looking toward the right side of the examinee (the left side in the drawing), and in this case, a cornea 111 of the right eye 11 has an elliptical shape. Therefore, a width W1 of the cornea 111 is smaller than a height H1.

The strabismus determination apparatus 2 of the present embodiment can determine the degree of strabismus based on the shapes of such corneas 111 and 121. Specifically, a value according to ratios of the widths W1 and W2 of the corneas 111 and 121 to the heights H1 and H2 of the corneas 111 and 121 (H1/W1, H2/W2) can be used as the degree of strabismus. In the present embodiment, it can be determined that the narrower the width of the cornea, the higher the degree of strabismus. Note that a magnitude of a difference between the width W1 of the cornea 111 of the right eye 11 and the width W2 of the cornea 121 of the left eye 12 may be used as the degree of strabismus. Also, a magnitude of a difference between widths (W1max, W2max) when the right eye 11 and the left eye 12 are each looking straight ahead and the widths (W1, W2) appearing in the captured image may be used as the degree of strabismus.

### Strabismus Determination Apparatus 2

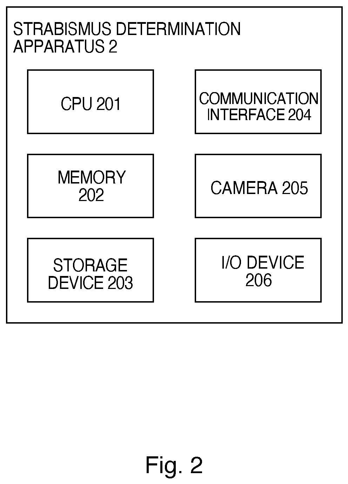

Fig. 2 is a diagram showing a hardware configuration example of the strabismus determination apparatus 2. Note that the illustrated configuration is an example, and the apparatus may have other configurations. The strabismus determination apparatus 2 includes a CPU 201, a memory 202, a storage device 203, a communication interface 204, a camera 205, and an input/output device 206. The storage device 203 is, for example, a hard disk drive, a solid state drive, a flash memory, or the like that stores various data and programs. The communication interface 204 is an interface for connecting to a communication network, and is, for example, an adapter for connecting to Ethernet (registered trademark), a modem for connecting to a public telephone network, a wireless communication device for performing wireless communication, a USB (Universal Serial Bus) connector or an RS232C connector for serial communication, or the like. The input/output device 206 is a device for inputting and outputting data, and is, for example, a touch panel display. The input/output device 206 can include a plurality of devices, and may include, for example, a keyboard, a mouse, a touch panel, buttons, a microphone, a display, a printer, a speaker, and the like. Note that each functional unit of the strabismus determination apparatus 2 described later is realized by the CPU 201 reading a program stored in the storage device 203 into the memory 202 and executing the program, and each storage unit of the strabismus determination apparatus 2 is realized as a part of a storage area provided by the memory 202 and the storage device 203.

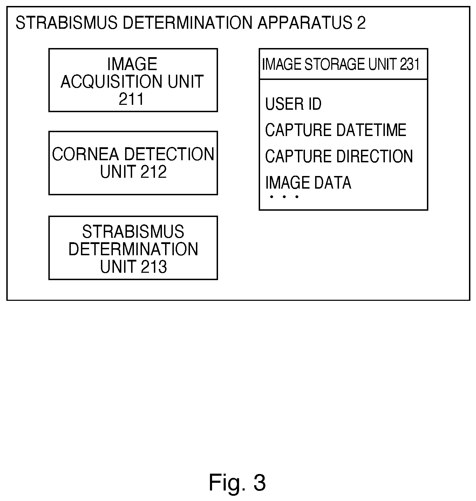

Fig. 3 is a diagram showing a software configuration example of the strabismus determination apparatus 2. The strabismus determination apparatus 2 includes an image acquisition unit 211, a cornea detection unit 212, a strabismus determination unit 213, and an image storage unit 231.

The image acquisition unit 211 acquires an image captured of the eyes of the examinee from the front of the examinee. The image acquisition unit 211 can, for example, output a message instructing a user to capture from the front of the face, and acquire an image captured by the user with the camera 205.

The image storage unit 231 stores the image acquired by the image acquisition unit 211. The image storage unit 231 can store image data in association with a user ID for identifying a user, a capture date and time, and a capture direction (for example, can be expressed as an angle centered on a surface of the face of the examinee (for example, the glabella), with the front of the examinee being 0 degrees).

The cornea detection unit 212 analyzes the image and detects a shape corresponding to the cornea from the image. The cornea detection unit 212 is configured to detect a shape surrounding a region corresponding to the cornea using a known image processing technique, and here, a detailed description of the processing will be omitted. In the present embodiment, the cornea detection unit 212 detects the cornea as (approximating to) a circle or an ellipse.

The strabismus determination unit 213 determines the degree of strabismus based on at least the width of the cornea. In the present embodiment, the strabismus determination unit 213 can determine the degree of strabismus according to a ratio of the width W of the cornea to the height H of the cornea. When the corneas 111 and 121 are detected, the strabismus determination unit 213 estimates an ellipse that approximates in shape to the corneas 111 and 121. A known method can be used for the ellipse estimation processing. The strabismus determination unit 213 can obtain the width W and the height H from the estimated ellipse. As a result, even when a part of the corneas 111 and 121 is occluded by an eyelid or the like, the height H can be accurately calculated. Note that only the height H may be obtained from the ellipse, and the width W may be obtained from the detected corneas 111 and 121.

Note that the strabismus determination unit 213 can also determine the degree of strabismus from only the width W. For example, the cornea detection unit 212 detects the corneas 111 and 121 of both the right eye and the left eye from the image, and the strabismus determination unit 213 can compare the width W1 of the right eye and the width W2 of the left eye, and determine, for example, a difference (absolute value) between the width W1 of the right eye and the width W2 of the left eye as the degree of strabismus. Also, the user can be instructed to move both eyes left and right, the cornea detection unit 212 can detect the corneas 111 and 121 from a video capturing a state of the eyes moving or still images captured at multiple time points of the state of the eyes moving, and the strabismus determination unit 213 can specify maximum values W1max and W2max of the width W1 of the right eye and the width W2 of the left eye, respectively. Subsequently, the user is instructed to look straight ahead, and capture is performed from the front of the face, the cornea detection unit 212 detects the corneas 111 and 121 from an image captured from the front of the user, and the strabismus determination unit 213 obtains the widths W1 and W2 of the detected corneas 111 and 121, and can determine the degree of strabismus according to an average value, the larger one, or the smaller one of differences (W1max-W1, W2max-W2) between the maximum values W1max and W2max and the widths W1 and W2 of the corneas in the image from the front.

### Operation

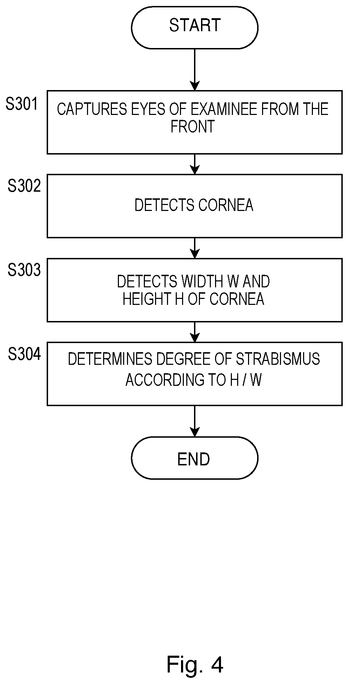

Fig. 4 is a diagram for explaining a flow of processing by the strabismus determination apparatus 2.

The strabismus determination apparatus 2 captures the eyes of the examinee from the front using the camera 205 (S301). Note that whether or not the capture is from the front can be left to the examinee, and the strabismus determination apparatus 2 itself may not determine whether or not the capture is from the front of the examinee. The strabismus determination apparatus 2 detects the eyes of the examinee from the captured image (S302). Only the right eye, only the left eye may be detected, or both eyes may be detected.

The strabismus determination apparatus 2 obtains the width W and the height H of the detected cornea (S303), and can determine the degree of strabismus according to a ratio of the width W to the height H (S304). Note that as described above, the degree of strabismus can also be determined according to a difference (W1-W2) between the widths W1 and W2 of the left and right corneas, or according to at least one of maximum values W1max and W2max (or at least one of them) of the width of the cornea when the eyes are moved left and right and a difference (W1max-W1, W2max-W2) between the widths W1 and W2 of the corneas in an image captured from the front, or according to an average value of the difference.

As described above, according to the strabismus determination apparatus 2 of the present embodiment, the degree of strabismus of the examinee can be determined only from a self-captured image of the examinee.

### Second Embodiment

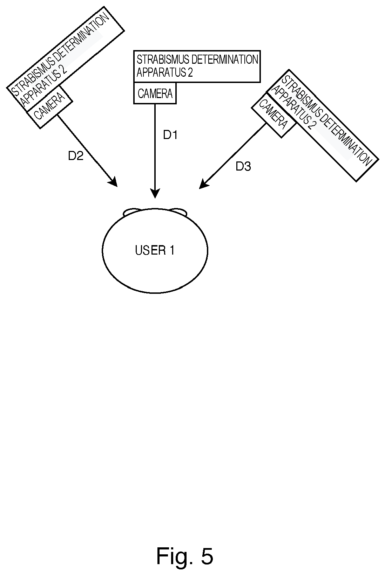

Fig. 5 is a diagram for explaining a state of capturing the examinee according to a second embodiment. In the second embodiment, not only capture in a direction D1 toward the front of the examinee, but also capture from directions D2 and/or D3 at an angle with respect to the front of the examinee is performed.

The image acquisition unit 211 acquires a first image (a captured image in a capture direction of D1) captured of the examinee from the front and a second image (a captured image in a capture direction of D2 and/or D3) captured at an angle with respect to the front of the examinee, the cornea detection unit 212 detects first and second corneas from the first and second images, and the strabismus determination unit 213 can determine the degree of strabismus based on the widths of the first and second corneas.

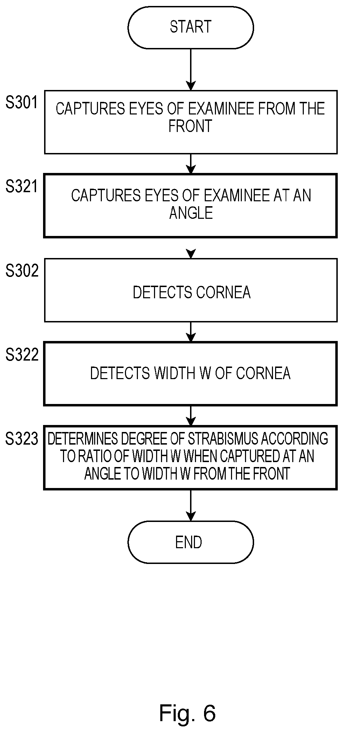

Fig. 6 is a diagram for explaining a flow of processing by the strabismus determination apparatus 2 according to the second embodiment. The strabismus determination apparatus 2 captures the eyes of the examinee from the front (S301), and further captures the eyes of the examinee at an angle (S321). The strabismus determination apparatus 2 detects the cornea of the examinee from the captured images (S302), and detects the width W of the cornea in each image (S322). The strabismus determination apparatus 2 can determine the degree of strabismus according to a ratio of the width W of the cornea when captured at an angle to the width W of the cornea when captured from the front (S323). In this case, for example, if the width W of the cornea in the second image captured in the capture direction D2 is larger than the width W of the cornea in the first image captured in the capture direction D1, it can be determined that the cornea is facing in a direction closer to a direction parallel to D2 than D1. The degree of strabismus can be determined according to the change in magnitude in this case.

### Third Embodiment

In the first and second embodiments, strabismus (exotropia or esotropia) in which gaze is deviated in the left-right direction was assumed, but in the third embodiment, strabismus (vertical strabismus) in which gaze is deviated in the vertical direction is determined.

In the third embodiment, a value according to ratios of the heights H1 and H2 of the corneas 111 and 121 to the widths W1 and W2 of the corneas 111 and 121 (W1/H1, W2/H2) can be used as the degree of vertical strabismus. In the third embodiment, it can be determined that the smaller the height of the cornea, the higher the degree of vertical strabismus. Note that a magnitude of a difference between the height H1 of the cornea 111 of the right eye 11 and the height H2 of the cornea 121 of the left eye 12 may be used as the degree of vertical strabismus. Also, a magnitude of a difference between heights (H1max, H2max) when the right eye 11 and the left eye 12 are each looking straight ahead and the heights (H1, H2) appearing in the captured image may be used as the degree of vertical strabismus.

In the third embodiment, the strabismus determination unit 213 determines the degree of vertical strabismus based on at least the height of the cornea. In the third embodiment, the strabismus determination unit 213 can determine the degree of strabismus according to a ratio of the height H of the cornea to the width W of the cornea. When the corneas 111 and 121 are detected, the strabismus determination unit 213 estimates an ellipse that approximates in shape to the corneas 111 and 121. A known method can be used for the ellipse estimation processing. The strabismus determination unit 213 can obtain the width W and the height H from the estimated ellipse. As a result, even when a part of the corneas 111 and 121 is occluded by an eyelid or the like, the width W and the height H can be accurately calculated.

Note that the strabismus determination unit 213 can also determine the degree of vertical strabismus from only the height H. For example, the cornea detection unit 212 detects the corneas 111 and 121 of both the right eye and the left eye from the image, and the strabismus determination unit 213 can compare the height H1 of the right eye and the height H2 of the left eye, and use, for example, a difference (absolute value) between the height H1 of the right eye and the height H2 of the left eye as the degree of vertical strabismus. Also, the user can be instructed to move both eyes up and down, the cornea detection unit 212 can detect the corneas 111 and 121 from a video capturing a state of the eyes moving or still images captured at multiple time points of the state of the eyes moving, and the strabismus determination unit 213 can specify maximum values H1max and H2max of the height H1 of the right eye and the height H2 of the left eye, respectively. Subsequently, the user is instructed to look straight ahead, and capture is performed from the front of the face, the cornea detection unit 212 detects the corneas 111 and 121 from an image captured from the front of the user, and the strabismus determination unit 213 obtains the heights H1 and H2 of the detected corneas 111 and 121, and can determine the degree of vertical strabismus according to an average value, the larger one, or the smaller one of differences (H1max-H1, H2max-H2) between the maximum values H1max and H2max and the heights H1 and H2 of the corneas in the image from the front.

### Fourth Embodiment

In the fourth embodiment, the degree of strabismus in both the left-right direction and the vertical direction is determined.

In the fourth embodiment, a value according to both a ratio of the heights H1 and H2 of the corneas 111 and 121 to the widths W1 and W2 of the corneas 111 and 121 (W1/H1, W2/H2) and a ratio of the widths W1 and W2 of the corneas 111 and 121 to the heights H1 and H2 of the corneas 111 and 121 (H1/W1, H2/W2) can be used as the degree of strabismus. In the fourth embodiment, it can be determined that the narrower the width of the cornea and the smaller the height of the cornea, the higher the degree of strabismus. Note that a magnitude of a difference between the width W1 and the height H1 of the cornea 111 of the right eye 11 and the width W2 and the height H2 of the cornea 121 of the left eye 12 may be used as the degree of strabismus. Also, using widths and heights (W1max, H1max, W2max, H2max) when the right eye 11 and the left eye 12 are each looking straight ahead and the widths and heights (W1, H1, W2, H2) appearing in the captured image, a value obtained by summing or weighted summing a width difference (W1max-W1, W2max-W2) and a height difference (H1max-H1, H2max-H2) can be used as the degree of strabismus.

Also in the fourth embodiment, similarly to the above-described embodiments, an ellipse approximating to the corneas 111 and 121 can be estimated, and the degree of strabismus can be calculated using the width W and the height H of the ellipse.

In the fourth embodiment, the strabismus determination unit 213 can also determine the degree of vertical strabismus from only the width W and the height H. For example, the cornea detection unit 212 detects the corneas 111 and 121 of both the right eye and the left eye from the image, and the strabismus determination unit 213 can compare the width W1 and the height H1 of the right eye and the width W2 and the height H2 of the left eye, and, for example, sum a difference (absolute value) between the width W1 of the right eye and the width W2 of the left eye and a difference (absolute value) between the height H1 of the right eye and the height H2 of the left eye to obtain the degree of strabismus.

Also, the user can be instructed to move both eyes up and down, the cornea detection unit 212 can detect the corneas 111 and 121 from a video capturing a state of the eyes moving or still images captured at multiple time points of the state of the eyes moving, and the strabismus determination unit 213 can specify maximum values H1max and H2max of the height H1 of the right eye and the height H2 of the left eye, respectively. Subsequently, the user is instructed to look straight ahead, and capture is performed from the front of the face, the cornea detection unit 212 detects the corneas 111 and 121 from an image captured from the front of the user, and the strabismus determination unit 213 obtains the heights H1 and H2 of the detected corneas 111 and 121, and can determine the degree of vertical strabismus according to an average value, the larger one, or the smaller one of differences (H1max-H1, H2max-H2) between the maximum values H1max and H2max and the heights H1 and H2 of the corneas in the image from the front.

Although the present embodiment has been described above, the above embodiment is for facilitating the understanding of the present invention, and is not for limiting and interpreting the present invention. The present invention can be modified and improved without departing from the spirit thereof, and the present invention also includes equivalents thereof.

For example, the degree of strabismus in the left-right direction and the degree of strabismus in the vertical direction may be calculated and output, respectively.

### Disclosure Items

Note that the present disclosure also includes the following configurations.

### Item 1

A strabismus determination apparatus comprising:
an image acquisition unit configured to acquire an image captured of eyes of an examinee from the front of the examinee;
a cornea detection unit configured to analyze the image and detect a shape corresponding to the cornea from the image; and
a strabismus determination unit configured to determine a degree of strabismus based on at least one of a width and a height of the cornea.

### Item 2

The strabismus determination apparatus according to item 1, wherein
the strabismus determination unit estimates an ellipse showing the shape of the cornea, and determines the degree of strabismus based on a width of the ellipse and a height of the ellipse.

### Item 3

The strabismus determination apparatus according to item 2, wherein
the strabismus determination unit determines the degree of strabismus according to at least one of a ratio of the width to the height and a ratio of the height to the width.

### Item 4

The strabismus determination apparatus according to item 1, wherein
a right eye and a left eye are captured in the image,
the cornea detection unit detects a first cornea of the right eye and a second cornea of the left eye from the image, and
the strabismus determination unit determines the degree of strabismus based on at least one of a width and a height of the first cornea and at least one of a width and a height of the second cornea.

### Item 5

The strabismus determination apparatus according to item 1, wherein
the image acquisition unit acquires a first image captured of the examinee from the front and a second image captured at an angle with respect to the front of the examinee,
the cornea detection unit detects first and second corneas from the first and second images, and
the strabismus determination unit determines the degree of strabismus based on at least one of widths and heights of the first and second corneas.

### Item 6

A strabismus determination method executed by an information processing apparatus, the method comprising:
acquiring an image captured of eyes of an examinee from a front of the examinee;
analyzing the image and detecting a shape corresponding to the cornea from the image; and
determining a degree of strabismus based on at least one of a width and a height of the cornea.

### Item 7

A program for causing an information processing apparatus to execute:
acquiring an image captured of eyes of an examinee from a front of the examinee;
analyzing the image and detecting a shape corresponding to the cornea from the image; and
determining a degree of strabismus based on at least one of a width and a height of the cornea.

### Reference Signs List

2 strabismus determination apparatus

## Claims

1. A strabismus determination apparatus comprising:
an image acquisition unit configured to acquire an image captured of a right eye and a left eye of an examinee from a front of the examinee;
a cornea detection unit configured to analyze the image and detect a cornea of the right eye and a cornea of the left eye from the image; and
a strabismus determination unit configured to determine a degree of strabismus based on a difference between at least one of a width and a height of the cornea of the right eye and at least one of a width and a height of the cornea of the left eye.

2. The strabismus determination apparatus according to claim 1, wherein:
the strabismus determination unit estimates an ellipse indicating a shape of each cornea of the right eye and the left eye, and determines the degree of strabismus according to a difference between at least one of a width of the ellipse and a height of the ellipse corresponding to the right eye and at least one of a width of the ellipse and a height of the ellipse corresponding to the left eye.

3. The strabismus determination apparatus according to claim 1, wherein:
the strabismus determination unit determines the degree of strabismus according to a difference between a ratio of the width of the cornea of the right eye to the height of the cornea of the right eye and a ratio of the width of the cornea of the left eye to the height of the cornea of the left eye.

4. The strabismus determination apparatus according to claim 1, wherein:
the image acquisition unit acquires a first said image captured of an examinee from a front and a second said image captured at an angle with respect to the front of the examinee,
the cornea detection unit detects first and second said corneas of the right eye and the left eye from the first and second images, and
the strabismus determination unit determines the degree of strabismus based on a difference between at least one of a width and a height of the first cornea of the right eye and at least one of a width and a height of the second cornea of the left eye.

5. A strabismus determination method executed by an information processing apparatus, the method comprising:
acquiring an image captured of a right eye and a left eye of an examinee from a front of the examinee;
analyzing the image and detecting a cornea of the right eye and a cornea of the left eye from the image; and
determining a degree of strabismus based on a difference between at least one of a width and a height of the cornea of the right eye and at least one of a width and a height of the cornea of the left eye.

6. A program for causing an information processing apparatus to execute:
acquiring an image captured of a right eye and a left eye of an examinee from a front of the examinee;
analyzing the image and detecting a cornea of the right eye and a cornea of the left eye from the image; and
determining a degree of strabismus based on a difference between at least one of a width and a height of the cornea of the right eye and at least one of a width and a height of the cornea of the left eye.
